# EUROPEAN PATENT SPECIFICATION

(11) **EP 0 487 284 B1**
(45) Date of publication and mention of the grant of the patent: **12.04.1995**
(21) Application number: 91310619.1
(22) Date of filing: 18.11.1991
(51) Int. Cl.: C07C 51/12, C07C 51/56, C07C 51/487, C07C 51/573

(54) **Removal of carbonyl impurities from a carbonylation process stream**
Entfernung von Carbonylverunreinigungen aus dem Carbonylierungsprozessstrom
Elimination d'impuretés carbonyles du courant du procédé de carbonylation

(30) Priority: 19.11.1990 US 615666
(43) Date of publication of application: 27.05.1992
(73) Proprietor: HOECHST CELANESE CORPORATION, Somerville, N.J. 08876 (US)
(72) Inventor: Torrence, G. Paull, Corpus Christi, Texas (US); Scates, Mark O., Pearland, Texas (US); Gibbs, Russel K., Jr., Houston, Texas (US)
(74) Representative: De Minvielle-Devaux, Ian Benedict Peter

(56) References cited:
- DE-B- 1 058 041
- US-A- 2 862 854
- US-A- 4 341 741
- US-A- 4 690 912

## Description

### BACKGROUND OF THE INVENTION

This invention relates to a novel process for the purification of acetic acid and/or acetic anhydride which have been formed by the carbonylation of methanol or methyl acetate in the presence of a Group VIII metal carbonylation catalyst. More specifically, this invention relates to a novel process for removing carbonyl impurities from acetic acid and/or acetic anhydride formed by Group VIII metal catalyzed carbonylation processes.

Among currently-employed processes for synthesizing acetic acid one of the most useful commercially is the catalyzed carbonylation of methanol with carbon monoxide as taught in U.S. 3,769,329 issued to Paulik et al on October 30, 1973. The carbonylation catalyst comprises rhodium, either dissolved or otherwise dispersed in a liquid reaction medium or else supported on an inert solid, along with a halogen-containing catalyst promoter as exemplified by methyl iodide. The rhodium can be introduced into the reaction system in any of many forms, and it is not relevant, if indeed it is possible, to identify the exact nature of the rhodium moiety within the active catalyst complex. Likewise, the nature of the halide promoter is not critical. The patentees disclose a very large number of suitable promoters, most of which are organic iodides. Most typically and usefully, the reaction is conducted with the catalyst being dissolved in a liquid reaction medium through which carbon monoxide gas is continuously bubbled.

An improvement in the prior-art process for the carbonylation of an alcohol to produce the carboxylic acid having one carbon atom more than the alcohol in the presence of a rhodium catalyst is disclosed in copending, commonly assigned application U.S. Serial No. 870,267, filed June 3, 1986 and European patent application 161,874; published November 21, 1985. As disclosed therein acetic acid is produced from methanol in a reaction medium comprising methyl acetate, methyl halide, especially methyl iodide, and rhodium present in a catalytically-effective concentration. The invention therein resides primarily in the discovery that catalyst stability and the productivity of the carbonylation reactor can be maintained at surprisingly high levels, even at very low water concentrations, i.e. 4 wt.% or less, in the reaction medium (despite the general industrial practice of maintaining approximately 14 wt.% or 15 wt.% water) by maintaining in the reaction medium, along with a catalytically-effective amount of rhodium, at least a finite concentration of water, methyl acetate and methyl iodide, a specified concentration of iodide ions over and above the iodide content which is present as methyl iodide or other organic iodide. The iodide ion is present as a simple salt, with lithium iodide being preferred. The applications teach that the concentration of methyl acetate and iodide salts are significant parameters in affecting the rate of carbonylation of methanol to produce acetic acid especially at low reactor water concentrations. By using relatively high concentrations of the methyl acetate and iodide salt, one obtains a surprising degree of catalyst stability and reactor productivity even when the liquid reaction medium contains water in concentrations as low as about 0.1 wt.%, so low that it can broadly be defined simply as "a finite concentration" of water. Furthermore, the reaction medium employed improves the stability of the rhodium catalyst, i.e. resistance to catalyst precipitation, especially during the product-recovery steps of the process wherein distillation for the purpose of recovering the acetic acid product tends to remove from the catalyst the carbon monoxide which in the environment maintained in the reaction vessel, is a ligand with stabilizing effect on the rhodium. U.S. Serial No. 870,256 is herein incorporated by reference.

Acetic anhydride may also be produced from the carbonylation of methyl acetate or dimethyl ether by the above described processes if the reactants are essentially water and methanol free.

The acetic acid which is formed by the carbonylation of methanol is converted to a high purity product by conventional means such as by a series of distillations. While it is possible in this way to obtain acetic acid of relatively high purity, the acetic acid product formed by the above-described low water carbonylation is frequently deficient with respect to the permanganate time owing to the presence therein of small proportions of residual impurities. Since a sufficient permanganate time is an important commercial test which the acid product must meet for many uses, the presence therein of such impurities that degrade permanganate time is highly objectionable. The removal of minute quantities of these impurities from the acetic acid by conventional distillation techniques is not commercially feasible.

Among the impurities which degrade permanganate time of the acetic acid are carbonyls and organic iodides. It has now been discovered by the present inventors that during the production of acetic acid by the carbonylation of methanol or methyl acetate in the presence of a finite amount of water, carbonyl impurities such as acetaldehyde, acetone, methyl ethyl ketone, butyraldehyde, crotonaldehyde, etc. are present and further react to form aldol condensation products and/or react with iodide catalyst promoters to form multi-carbon alkyl iodides, i.e., ethyl iodide, butyl iodide, and hexyl iodide. In the formation of acetic anhydride or in acetic acid/acetic anhydride co-production units by carbonylation of methyl acetate and dimethyl ether, it is known that undesirable high boiling tars are formed in the catalyst solution. The tars are believed to be formed by aldol condensation of the carbonyl impurities such as acetaldehyde and acetone as well as by reaction of the carbonyl and aldol products with the formed acetic anhydride. The tars if not removed or sufficiently reduced will greatly diminish the catalyst activity, eventually resulting in the termination of the carbonylation reaction. Thus, not only has the precipitated tar become an environmental problem, the operation of the commercial carbonylation process has been degraded and made more costly as makeup catalyst is required. Several prior art inventions have dealt with the removal of the by-products, of particular note are Erpenbach et al, U. S. 4,717,454; Hoch and Wan, U. S. 4,252,748; and Larkin's, U. S. 4,434,241.

Unfortunately, it is difficult to completely remove the minor amounts of carbonyl impurities which are present by conventional means such as distillation inasmuch as the impurities have boiling points close to that of the acetic acid and acetic anhydride products. It is known to remove carbonyl impurities, in general, from organic streams by treating the organic streams with an amino compound which reacts with the carbonyls to form oximes followed by distillation to separate the purified organic product from the oxime reaction products. However, the additional treatment of the final product adds cost to the process and it has been found that distillation of the treated acetic acid product can result in additional impurities being formed. For example, it has been found that the formation of nitriles from the oximes readily occurs during distillation to remove the oximes. Obviously, if the final product is again contaminated, such process is not readily useful.

Thus, while removing carbonyls from the acetic acid and/or acetic anhydride carbonylation product has been found to be of critical importance to yield a pure product, it is necessary to determine where in the carbonylation process such materials can be removed and by what process without risk of further contamination.

### SUMMARY OF THE INVENTION

The process of the present invention is directed to the minimization of circulating carbonyl-containing and unsaturated organic materials in the reaction mixture thus resulting in a more facile purification of acetic acid and/or acetic anhydride which have been formed by the carbonylation of methanol, dimethyl ether, or methyl acetate in the presence of a Group VIII metal carbonylation catalyst. Such carbonylation reactions comprise catalytic reaction with carbon monoxide in the presence of a halide promoter such as an organic halide as disclosed in U.S. 3,769,329 or under low water conditions such as disclosed in aforementioned U.S. Serial No. 870,267 wherein the catalyst solution not only contains the Group VIII metal catalyst and organic halide promoter, but also contains an additional iodide salt. In such processes, a feed of methanol or methyl acetate is carbonylated in a liquid phase carbonylation reactor. Separation of products is achieved by directing the contents of a reactor to a flasher wherein the catalyst solution is withdrawn as a base stream and recycled to the reactor while the overhead which comprises largely the product acetic acid and/or anhydride along with methyl iodide, methyl acetate, and water (if only acetic anhydride is produced) is directed to a methyl iodide-acetic acid splitter column. The overhead from the splitter column comprises mainly organic iodides and methyl acetate whereas from the base of the splitter column is drawn the acetic acid or anhydride product which is usually directed to further purification by finishing distillation. The overhead from the splitter column containing the organic iodides is recycled to the carbonylation reactor. It has now been discovered that the carbonyl impurities which are found in the acetic acid product and which have been found to result in the formation of tars during the formation of acetic anhydride or in the co-production of acetic acid and acetic anhydride concentrate in the overhead from the splitter column. In accordance with the process of the present invention, the splitter column overhead is treated with a compound which reacts with the carbonyls to allow such carbonyls to be separated from the remaining overhead by means of distillation. Modified by such a treatment, the carbonylation of methanol yields an acetic acid product which has greatly improved permanganate time and is substantially free from carbonyl impurities. Moreover, in the carbonylation of methyl acetate to acetic anhydride, the formation of tars which has been found in commercial production units can be substantially reduced.

### DETAILED DESCRIPTION

The purification process of the present invention is useful in any process used to carbonylate methanol, dimethyl ether, or methyl acetate to acetic acid and/or acetic anhydride in the presence of a Group VIII metal catalyst such as rhodium and an iodide promoter. A particularly useful process is the low water rhodium catalyzed carbonylation of methanol to acetic acid as exemplified in aforementioned U.S. Serial No. 870,267. Generally, the rhodium component of the catalyst system is believed to be present in the form of a coordination compound of rhodium with a halogen component providing at least one of the ligands of such coordination compound. In addition to the coordination of rhodium and halogen, it is also believed that carbon monoxide ligands form coordination compounds or complexes with rhodium. The rhodium component of the catalyst system may be provided by introducing into the reaction zone rhodium in the form of rhodium metal, rhodium salts and oxides, organic rhodium compounds, coordination compounds of rhodium, and the like.

The halogen promoting component of the catalyst system consists of a halogen compound comprising an organic halide. Thus, alkyl, aryl, and substituted alkyl or aryl halides can be used. Preferably, the halide promoter is present in the form of an alkyl halide in which the alkyl radical corresponds to the alkyl radical of the feed alcohol which is carbonylated. Thus, in the carbonylation of methanol to acetic acid, the halide promoter will comprise methyl halide, and more preferably methyl iodide.

The liquid reaction medium employed may include any solvent compatible with the catalyst system and may include pure alcohols, or mixtures of the alcohol feedstock and/or the desired carboxylic acid and/or esters of these two compounds. The preferred solvent and liquid reaction medium for the low water carbonylation process comprises the carboxylic acid product. Thus, in the carbonylation of methanol to acetic acid, the preferred solvent is acetic acid.

When the reaction is used to manufacture acetic acid, water is contained in the reaction medium, but, at concentrations well below that which has heretofore been thought practical for achieving sufficient reaction rates. It is known that in rhodium-catalyzed carbonylation reactions of the type set forth in this invention, the addition of water exerts a beneficial effect upon the reaction rate (U.S. Patent No. 3,769,329). Thus, commercial operations run at water concentrations of at least 14 wt.% (EP 055618). Accordingly, it is quite unexpected that reaction rates substantially equal to and above reaction rates obtained with such high levels of water concentration can be achieved with water concentrations below 14 wt.% and as low as 0.1 wt.%.

In accordance with the carbonylation process most useful to manufacture acetic acid according to the present invention, the desired reaction rates are obtained even at low water concentrations by including in the reaction medium methyl acetate and an additional iodide ion which is over and above the iodide which is present as a catalyst promoter such as methyl iodide or other organic iodide. The additional iodide promoter is an iodide salt, with lithium iodide being preferred. It has been found that under low water concentrations, methyl acetate and lithium iodide act as rate promoters only when relatively high concentrations of each of these components are present and that the promotion is higher when both of these components are present simultaneously. This has not been recognized in the prior art previous to disclosure of commonly assigned U.S. Serial No. 870,267. The concentration of lithium iodide used in the reaction medium of the preferred carbonylation reaction system is believed to be quite high as compared with what little prior art there is dealing with the use of halide salts in reaction systems of this sort.

The carbonylation reaction of methanol to acetic acid product may be carried out by intimately contacting the methanol feed, which is in the liquid phase, with gaseous carbon monoxide bubbled through a liquid reaction medium containing the rhodium catalyst, methyl iodide promoting component, methyl acetate, and additional soluble iodide salt promoter, at conditions of temperature and pressure suitable to form the carbonylation product. It will be generally recognized that it is the concentration of iodide ion in the catalyst system that is important and not the cation associated with the iodide, and that at a given molar concentration of iodide the nature of the cation is not as significant as the effect of the iodide concentration. Any metal iodide salt, or any iodide salt of any organic cation, can be used provided that the salt is sufficiently soluble in the reaction medium to provide the desired level of the iodide. The iodide salt can be a quaternary salt of an organic cation or the iodide salt of an inorganic cation. Preferably it is an iodide salt of a member of the group consisting of the metals of Group Ia and Group IIa of the periodic table as set forth in the "Handbook of Chemistry and Physics" published by CRC Press, Cleveland, Ohio, 1975-76 (56th edition). In particular, alkali metal iodides are useful, with lithium iodide being preferred. In the low water carbonylation most useful in this invention, the additional iodide over and above the organic iodide promoter is present in the catalyst solution in amounts of from about 2 to about 20 wt.%, preferably 5-15 wt.%, the methyl acetate is present in amounts of from about 0.5 to about 30 wt.%, preferably 2-5 wt.%, and the methyl iodide is present in amounts of from about 5 to about 20 wt.%, preferably 10-16 wt.%, and most preferably 12-15 wt.%. The rhodium catalyst is present in amounts of from 200-1000 and preferably 300-600 ppm.

Typical reaction temperatures for carbonylation will be approximately 150-250°C, with the temperature range of about 180-220°C being the preferred range. The carbon monoxide partial pressure in the reactor can vary widely but is typically about 2-30 atmospheres, and preferably, about 3-10 atmospheres. Because of the partial pressure of by-products and the vapor pressure of the contained liquids, the total reactor pressure will range from about 15 to 40 atmospheres.

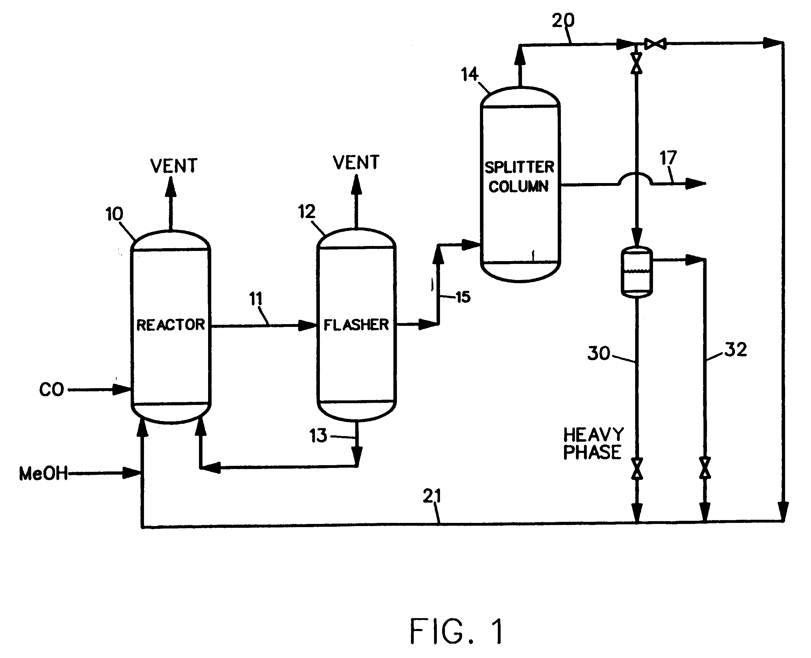

A reaction and acetic acid recovery system which is used for the iodide-promoted rhodium catalyzed carbonylation of methanol to acetic acid is shown in Figure 1 and comprises a liquid-phase carbonylation reactor 10, flasher 12, and a methyl iodide-acetic acid splitter column 14. The carbonylation reactor 10 is typically a stirred autoclave within which the reacting liquid contents are maintained automatically at a constant level. Into this reactor there are continuously introduced fresh methanol, sufficient water as needed to maintain at least a finite concentration of water in the reaction medium, recycled catalyst solution from the flasher base, a recycled methyl iodide and methyl acetate phase, and an aqueous acetic acid phase from the overhead of the methyl iodide-acetic acid splitter column 14. Alternate distillation systems can be employed so long as they provide means for recovering the crude acetic acid and recycling catalyst solution, methyl iodide, and methyl acetate to the reactor. In the preferred process, carbon monoxide is continuously introduced into the carbonylation reactor 10 just below the agitator which is used to stir the contents. The gaseous feed is, of course, thoroughly dispersed through the reacting liquid by this means. A gaseous purge stream is vented from the reactor to prevent buildup of gaseous by-products and to maintain a set carbon monoxide partial pressure at a given total reactor pressure. The temperature of the reactor is controlled automatically, and the carbon monoxide feed is introduced at a rate sufficient to maintain the desired total reactor pressure.

Liquid product is drawn off from carbonylation reactor 10 at a rate sufficient to maintain a constant level therein and is introduced to flasher 12 at a point intermediate between the top and bottom thereof via line 11. In flasher 12 the catalyst solution is withdrawn as a base stream 13 (predominantly acetic acid containing the rhodium and the iodide salt along with lesser quantities of methyl acetate, methyl iodide, and water), while the overhead 15 of the flasher comprises largely the product acetic acid along with methyl iodide, methyl acetate, and water. A portion of the carbon monoxide along with gaseous by-products such as methane, hydrogen, and carbon dioxide exits the top of the flasher.

The product acetic acid drawn from the side of methyl iodide-acetic acid splitter column 14 near the base (it can also be withdrawn as a base stream) is directed via line 17 for final purification such as to remove water as desired by methods which are obvious to those skilled in the art including, most preferably, distillation. The overhead 20 from methyl iodide-acetic acid splitter, comprising mainly methyl iodide and methyl acetate plus some water and acetic acid, is recycled via line 21 to the carbonylation reactor 10. When overhead 20 is condensed it typically splits into two liquid phases if sufficient water is present. The heavy phase 30 is comprised mainly of methyl iodide plus some methyl acetate and acetic acid. The light phase 32 is comprised mainly of water and acetic acid plus some methyl acetate. The overhead heavy phase 30 from methyl iodide-acetic acid splitter or the total overhead 20 if it does not separate is subject to treatment or these streams can be combined with recycle products from further purification processes containing methyl iodide, methyl acetate, water, and other impurities to become recycle 21 which is also subject to treatment according to this invention.

In accordance with the carbonylation process of the present invention, it has been found that carbonyl impurities which accumulate in the methyl iodide-rich phase 30 or into the total overhead 20 if it does not separate into two phases and are removed from this stream in the carbonylation process to yield a substantial improvement in acetic acid and/or anhydride product quality. Thus, the methyl iodide-rich phase 30 which contains carbonyl impurities such as acetaldehyde, crotonaldehyde, butyraldehyde, and the like, is reacted with a compound which converts the carbonyl impurities to derivatives which can be separated from the reaction product by distillation to provide a recycle stream free from carbonyl impurities. In the preferred embodiment, the methyl iodide-rich phase is treated with an aqueous amino compound. A subsequent separation is carried out to remove the nitrogenous-containing derivatives so as to minimize any conversion of the nitrogenous aldehyde derivatives to nitriles which can contaminate the purified recycle stream. Preferably the separation is a distillation to remove the volatile overhead from the non-volatile amine residues.

In the first stage of the preferred process, the combined recycle stream 30 which contains carbonyl impurities including aldeydes, such as acetaldehyde, crotonaldehyde, and butyraldehyde, is contacted with an amino compound, preferably an aqueous hydroxylamine salt, e.g., hydroxylamine hydrochloride, hydroxylamine sulfate, hydroxylamine bisulfate, or hydroxylamine phosphate. Since hydroxylamine slowly decomposes in its free form, it is commercially supplied as its acid salt. The free hydroxylamine is liberated upon treatment of the acid salt with a base such as potassium hydroxide, sodium hydroxide or lithium hydroxide. If sodium hydroxide is used as the base to liberate the hydroxylamine from its acidic sulfate salt, then such liberation also produces sodium sulfate as a byproduct. If a large amount of acetic acid or acetic anhydride remains in this stream 30, it may be removed, as for example, by distillation, prior to the addition of the amino compound.

The base should be used in an amount, for example, of about 0.5 equivalents per equivalent of starting hydroxylamine plus any amount needed for the neutralization of the residual acetic acid or acetic anhydride in the stream. The base is preferably used in an amount of 0.8-1.0 equivalents per equivalent of starting hydroxylamine so that a small amount of hydroxylamine remains in the form of its acid salt to create a pH buffer that maintains the pH of the reactant solution in the range of 4.0 to 7.0. Use of larger amounts of base can cause the pH to rise above 7 and result in the decomposition of the unstable hydroxylamine free base to undesirable volatile by-products such as ammonia initiating undesirable condensation reactions of the methyl iodide-rich combined recycle streams with the free hydroxylamine which is formed. The hydroxylamine acid salt is preferably used in an amount of 1 to 2 equivalents of starting hydroxylamine per equivalent of the carbonyl impurities which are contained in the combined recycle material 30. The amount of carbonyl impurities can be determined by analytical methods prior to reaction. It is also important that the pH of the reaction solution remains at or near pH 4.5 to maximize the oximation reaction. The reaction is run at a temperature of about 0° to 70°C for a period of from about 1 min. to 1 hour. Any pressure may be used and is not critical in the process.

Although hydroxylamine is the preferred amino compound for use in the process of this invention, other amino compounds are suitable including aniline and acid salts thereof such as aniline acetate, aniline sulphate, hydrazine, phenylhydrazine; alkyl amines such as methylamine, ethylamine propylamine, phenylamine and naphthylamine. Moreover, in less preferred embodiments, other compounds can be used to treat the splitter column overhead, including bisulfite salts, as for example sodium bisulfite.

Regardless of the type of amino compound used, nitrile formation from the reaction product of an aldehyde and amino compound can result during prolonged heating such as during distillation. Reaction of hydroxylamine and aldehydes yields an oxime whereas reaction with hydrazine yields the hydrazone. The nitrile forming reactions are shown below for (1) oxime products and (2) hydrazone products.

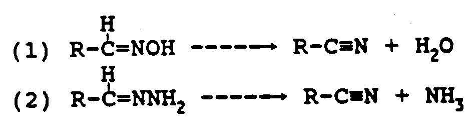

Subsequent to the addition of amino compound and reaction thereof with the carbonyl impurities, it is necessary to separate the recycle from the nitrogenous products from the treated stream before the stream is returned to the reactor. In accordance with the present invention, a series of steps are utilized to provide this separation and yield a purified recycle stream and, in particular, a pure recycle stream which is free from nitrile. Unfortunately, typical distillation procedures which are used to separate purified organic streams from the nitrogenous products formed by reaction of the aldehyde impurities and the amino compound as in the prior art tend to produce nitriles by the reaction schemes described above upon prolonged heating.

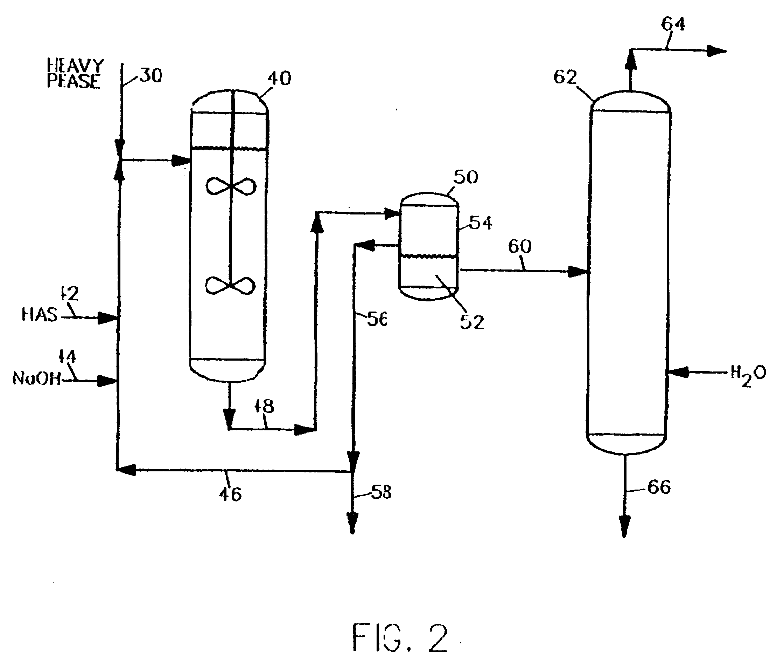

The separation of the pure recycle stream from the impure nitrogenous reaction product can be more readily described by referring to Figure 2 which is a schematic of the recycle purification process of the present invention. In Figure 2 and following example, hydroxylamine is used as the amino compound. It is to be understood that any reactive amino compound is useful in the process of this invention and, thus, the description below is not intended to limit the invention. Thus, referring to Figure 2, it can be seen that entering reactor 40 is recycle stream 30, hydroxylamine sulfate (HAS) feedstream 42, and sodium hydroxide feedstream 44, as well as recycle aqueous stream 46. The reaction takes place in reactor 40 as described above in which the carbonyl impurities contained in the recycle stream are reacted with hydroxylamine to form oximation products which are soluble in the aqueous phase. The reactor can be of any suitable equipment known in the art including a stirred back-mix or plug flow reactor.

Subsequent to reaction, the reaction products are collected via line 48 from reactor 40 and directed to decanter 50 for separation of the purified recycle 52 from the light aqueous phase 54 which contains unreacted hydroxylamine sulfate as well as most of the oximation products from reaction of the carbonyl impurities in recycle 30 with the hydroxylamine. The aqueous phase containing the hydroxylamine sulfate may be fully or partially recycled to reactor 40 via lines 56 and 46 or partially purged via line 58. The recirculation of the aqueous phase greatly improves pH control which is necessary to release the hydroxylamine from the hydroxylamine salt and allows optimum reaction with the carbonyl impurities. Recirculation of the aqueous phase also minimizes usage of hydroxylamine. The organic phase 52 containing methyl iodide-rich recycle, minor amounts of water as well as trace amounts of hydroxylamine sulfate, oximes and impurities which do not separate with the aqueous hydroxylamine sulfate phase is withdrawn from the decanter 50 via line 60 and directed to distillation tower 62 for removal of these components from the recycle. Upon distillation in tower 62, a distillate containing a purified recycle stream leaves the tower via line 64. This light ends stream can be recycled to carbonylation reactor 10 via line 21 as previously described. The bottoms 66 from distillation tower 62 comprises the separated aqueous oximes as well as other impurities such as alkanes.

It is important to reduce the oxime content at the top of distillation tower 62. We have found that oximes such as those formed by reaction of the hydroxyl amine and aldehydes, in particular, acetaldehyde oxime can readily convert to the nitrile, e.g., acetonitrile, which has a boiling point close to the methyl iodide-rich recycle and which will distill with and contaminate the recycle phase distillate 64 leaving distillation tower 62. Such conversion occurs more readily under conditions of high temperature in an acidic medium. Accordingly, in order to remove any oxime as well as nitrile from distillate 64 leaving distillation tower 62, additional water must be added to the distillation tower 62. The water content added should be in an amount, for example, 0.1 - 3 feed volume ratio of water to organic phase (tower) feed 60. The water partitions the oxime to the bottom of distillation tower 62, and reduces the temperature needed for distillation, further reducing the undesirable nitrile formation.

In order to demonstrate the value of removing the carbonyl products from the methyl iodide rich phase 30, the following experiments were performed. During operation of a commercial unit manufacturing acetic acid the composition of the contents of line 30 was modified. A charge of 500 pounds of acetaldehyde was injected into line 30 in less than one hour. Analysis was performed on several product purification streams. At the point of addition the base level of acetaldehyde and condensation products thereof increased from a base level of 1.5% to 2.0% at the 7 hour time point. At line 21 the level of acetaldehyde products increased from a base level of 0.6% to a maximum of 1.5% in four hours. In the vent from the flasher the aldehyde level increased to a maximum of 0.6% within 1 hour and took 15 hours to return to the base level. In the reactor 10 the aldol products reached a peak of 0.27% in 10 hours and took another 10 hours to return to the base level of 0.2%. The acetaldehyde derived products may include one or more of the following compounds: acetaldehyde, acetaldehyde dimethyl acetal, crotonaldehyde, butyraldehyde, 2-ethyl crotonaldehyde, 2-ethyl butyraldehyde, and 2-hexenal. Analysis of the final product stream during the 24 hour period following acetaldehyde addition showed a 2-fold increase in concentration in each of the following by-products: crotonaldehyde, ethyl crotonaldehyde, and butyl acetate.

### EXAMPLE 1

A continuous process as illustrated in Figure 2 was used to remove acetaldehyde from a process stream of primarily methyl iodide that contains 3909 ppm acetaldehyde.
Operating conditions were:

| At reactor 40 | |
|---|---|
| Methyl iodide stream flow to reactor (30) | 6.6 gm/min |
| 30% aqueous hydroxylamine flow to reactor (42) | 0.16 gm/min |
| 30% aqueous sodium hydroxide flow to reactor (44) | 0.06 gm/min |
| Reactor purge flow | 0.31 gm/min |
| Reactor aqueous phase recycle flow to reactor | 6.3 ml/min |
| Reactor aqueous phase recycle pH | 4.7 |

| At Tower 62 | |
|---|---|
| Water feed flow to Tower | 3.2 gm/min |
| Tower distillate flow (64) | 6.5 gm/min |
| Tower bottoms aqueous flow (66) | 3.2 gm/min |
| Tower reflux/distillate ratio | 2.0 |
| Tower methyl iodide stream feed temperature | 39 deg C |
| Tower water feed temperature | 52 deg C |
| Tower base temperature | 96 deg C |
| 20 tray column (62) with reactor organic phase (52) column feed (60) on tray 15 and water column feed on tray (5) | |

The tower distillate product stream (64) of purified methyl iodide was analyzed by gas chromatography and contained only 573 ppm acetaldehyde. This result demonstrates a significant reduction in the acetaldehyde concentration in the methyl iodide process stream (30).

### COMPARATIVE EXAMPLE

Operating conditions were:

| At reactor 40 | |
|---|---|
| Methyl iodide stream flow to reactor (30) | 5.20 gm/min |
| 30% aqueous hydroxylamine flow to reactor (42) | 0 gm/min |
| 30% aqueous sodium hydroxide flow to reactor (44) | 0 gm/min |
| Reactor purge flow | 0.23 gm/min |
| Reactor aqueous phase recycle flow to reactor | 6.8 ml/min |
| Reactor aqueous phase recycle pH | 4.6 |

| At Tower 62 | |
|---|---|
| Water feed flow to Tower | 3.0 gm/min |
| Tower distillate flow (64) | 5.0 gm/min |
| Tower bottoms aqueous flow (66) | 3.2 gm/min |
| Tower reflux/distillate ratio | 2.0 gm/min |
| Tower methyl iodide stream feed temperature | 39 deg C |
| Tower water feed temperature | 54 deg C |
| Tower base temperature | 96 deg C |
| 20 tray column (62) with reactor organic phase (52) column feed (60) on tray 15 and water column feed on tray (5). | |

The tower distillate product stream (64) of purified methyl iodide was analyzed by gas chromatography and contained 2884 ppm acetaldehyde. This result demonstrates a slight reduction in the acetaldehyde concentration in the methyl iodide process stream (30). This reduction of the acetaldehyde concentration is not from chemical reactive treatment of acetaldehyde, but only a result from water extraction of acetaldehyde from the reactor organic phase (52) into the reactor aqueous phase (54) during mixing in the reactor.

## Claims

1. In a process for the carbonylation of one or more compounds of the group consisting of methanol, dimethyl ether, or methyl acetate to a product of acetic acid, acetic anhydride or both wherein said methanol, dimethyl ether, or methyl acetate is carbonylated in a reaction medium containing a Group VIII metal carbonylation catalyst and methyl iodide, the products of said carbonylation separated into a volatile phase containing said product, unreacted dimethyl ether or methyl acetate and methyl iodide and a less volatile phase comprising said Group VIII metal catalyst, said volatile phase distilled to yield a product and an overhead containing unreacted methanol, dimethyl ether, or methyl acetate and methyl iodide, and recycling said overhead to said carbonylation reactor, the improvement which comprises:
contacting said overhead with an aqueous solution of an amino compound under conditions at which said amino compound reacts with carbonyl impurities in said overhead for conversion to water soluble nitrogenous derivatives of said carbonyls, subsequently forming an organic phase containing a major portion of said dimethyl ether or methyl acetate, methyl iodide, and an aqueous phase containing said nitrogenous derivatives, separating said organic phase from said aqueous phase to yield a purified material for recycle to the reactor.

2. In a process for the carbonylation of methanol, dimethyl ether, or methyl acetate to a product of acetic acid, acetic anhydride or both wherein said methanol, dimethyl ether, or methyl acetate is carbonylated in a reaction medium containing a Group VIII metal carbonylation catalyst and an organic iodide catalyst promoter, the products of said carbonylation separated into a volatile phase containing said product, unreacted methanol, dimethyl ether, or methyl acetate and organic iodide and a less volatile phase comprising said Group VIII metal catalyst, said volatile phase distilled to yield a product phase and an overhead containing unreacted methanol, dimethyl ether, or methyl acetate and organic iodide, and recycling said overhead to said carbonylation reactor, the improvement which comprises:
contacting said overhead with a compound under conditions at which said compound reacts with carbonyl impurities in said overhead for conversion to derivatives of said carbonyls, and subsequently distilling said overhead to separate a purified overhead for said recycle from said carbonyl derivative.

3. The process of claim 2 wherein said compound is an aqueous amino compound which forms water soluble nitrogenous derivatives of said carbonyls, said process further comprising prior to distillation forming an organic phase containing a major portion of said methanol, dimethyl ether, or methyl acetate and organic iodide and an aqueous phase containing said nitrogenous derivatives, distilling said organic phase to separate a purified overhead for said recycle.

4. The process of claim 2 or 3 wherein said organic iodide catalyst promoter is methyl iodide.

5. The process of claim 1, 3 or 4 wherein said amino compound is hydroxyl amine and said nitrogenous derivatives are oximes.

6. The process of claim 5 wherein said amino compound comprises a hydroxylamine acid salt and a base to liberate said hydroxylamine from said salt.

7. The process of claim 6 wherein said hydroxylamine salt is hydroxylamine sulfate.

8. The process of claim 6 or 7 wherein said base is sodium hydroxide.

9. The process of any of claims 1-8 wherein water is added to the said separation or distillation of said organic phase to provide a water content in an amount of 0.1 - 3 feed volume ratio of water to organic phase so as to reduce the concentration of nitrogenous derivatives in the purified overhead.

10. The process of claim 9 wherein said aqueous solution of an amino compound comprises a hydroxylamine salt and a base to liberate said hydroxylamine from said salt.

11. The process of any of claims 1-10 wherein methyl acetate or dimethyl ether is carbonylated to a product consisting essentially of acetic anhydride.

12. The process of any of claims 1-10 wherein methyl acetate or dimethyl ether is carbonylated to a product comprising both acetic acid and acetic anhydride.

13. The process of any of claims 1-10 wherein methanol or methyl acetate is carbonylated to a product consisting essentially of acetic acid.

14. The process of any of claims 1-10 wherein methanol or methyl acetate is carbonylated to a product comprising both acetic acid and acetic anhydride.

15. The process of any of claims 1-14 wherein said carbonyl impurities present in said overhead comprise acetaldehyde.

16. The process of any of claims 1-14 wherein said carbonyl impurities present in said overhead comprise ketones.

17. The process of claim 16 wherein said ketones include acetone.

## Patentansprüche

1. Verfahren zur Carbonylierung einer oder mehrerer Verbindungen aus der Gruppe, die aus Methanol, Dimethylether oder Methylacetat besteht, zu einem Produkt von Essigsäure, Essigsäureanhydrid oder beiden, wobei das Methanol, der Dimethylether oder das Methylacetat in einem Reaktionsmedium carbonyliert wird, das ein Metall der Gruppe VIII als Carbonylierungskatalysator und Methyliodid enthält, die Produkte der Carbonylierung in eine flüchtige Phase, die das Produkt, nicht umgesetzten Dimethylether oder Methylacetat und Methyliodid enthält, und eine weniger flüchtige Phase, die den Metallkatalysator der Gruppe VIII umfaßt, aufgetrennt werden, die flüchtige Phase destilliert wird, was ein Produkt und einen Vorlauf, der nicht umgesetztes Methanol, Dimethylether oder Methylacetat und Methyliodid enthält, ergibt, und der Vorlauf in den Carbonylierungsreaktor zurückgeführt wird, dadurch gekennzeichnet, daß das Verfahren folgendes umfaßt:
In-Kontakt-Bringen des Vorlaufs mit einer wäßrigen Lösung einer Aminoverbindung unter Bedingungen, bei denen die Aminoverbindung mit Carbonylverunreinigungen in dem Vorlauf unter Umwandlung zu wasserlöslichen Stickstoffderivaten der Carbonylverbindungen reagiert, anschließend Bilden einer organischen Phase, die einen größeren Teil des Dimethylethers oder Methylacetats und Methyliodid enthält, und einer wäßrigen Phase, die die Stickstoffderivate enthält, Trennen der organischen Phase von der wäßrigen Phase, was ein gereinigtes Material zur Rückführung in den Reaktor ergibt.

2. Verfahren zur Carbonylierung von Methanol, Dimethylether oder Methylacetat zu einem Produkt von Essigsäure, Essigsäureanhydrid oder beiden, wobei das Methanol, der Dimethylether oder das Methylacetat in einem Reaktionsmedium carbonyliert wird, das ein Metall der Gruppe VIII als Carbonylierungskatalysator und ein organisches Iodid als Katalysator-Promotor enthält, die Produkte der Carbonylierung in eine flüchtige Phase, die das Produkt, nicht umgesetztes Methanol, Dimethylether oder Methylacetat und organisches Iodid enthält, und eine weniger flüchtige Phase, die den Metallkatalysator der Gruppe VIII umfaßt, aufgetrennt werden, die flüchtige Phase destilliert wird, was eine Produktphase und einen Vorlauf, der nicht umgesetztes Methanol, Dimethylether oder Methylacetat und organisches Iodid enthält, ergibt, und der Vorlauf in den Carbonylierungsreaktor zurückgeführt wird, dadurch gekennzeichnet, daß das Verfahren folgendes umfaßt:
In-Kontakt-Bringen des Vorlaufs mit einer Verbindung unter Bedingungen, bei denen die Verbindung mit Carbonylverunreinigungen in dem Vorlauf unter Umwandlung zu Derivaten der Carbonylverbindungen reagiert, und anschließend Destillieren des Vorlaufs unter Abtrennen eines gereinigten Vorlaufs von dem Carbonylderivat für die Rückführung.

3. Verfahren gemäß Anspruch 2, wobei die Verbindung eine wäßrige Aminoverbindung ist, die wasserlösliche Stickstoffderivate der Carbonyle bildet, wobei das Verfahren weiterhin vor der Destillation die Bildung einer organischen Phase, die einen größeren Teil des Methanols, Dimethylethers oder Methylacetats und organisches Iodid enthält, und einer wäßrigen Phase, die die Stickstoffderivate enthält, umfaßt, Destillieren der organischen Phase unter Antrennen eines gereinigten Vorlaufs für die Rückführung.

4. Verfahren gemäß Anspruch 2 oder 3, wobei das organische Iodid als Katalysator-Promotor Methyliodid ist.

5. Verfahren gemäß Anspruch 1, 3 oder 4, wobei die Aminoverbindung Hydroxylamin ist und die Stickstoffderivate Oxime sind.

6. Verfahren gemäß Anspruch 5, wobei die Aminoverbindung ein Hydroxylamin-Säuresalz und eine Base zum Freisetzen des Hydroxylamins aus dem Salz umfaßt.

7. Verfahren gemäß Anspruch 6, wobei das Hydroxylaminsalz Hydroxylaminsulfat ist.

8. Verfahren gemäß Anspruch 6 oder 7, wobei die Base Natriumhydroxid ist.

9. Verfahren gemäß einem der Ansprüche 1-8, wobei Wasser zu der Auftrennung oder Destillation der organischen Phase hinzugegeben wird, so daß ein Wassergehalt in einer Menge von 0,1-3 Einspeisungsvolumenverhältnis Wasser zu organischer Phase erhalten wird, um die Konzentration von Stickstoffderivaten in dem gereinigten Vorlauf zu reduzieren.

10. Verfahren gemäß Anspruch 9, wobei die wäßrige Lösung einer Aminoverbindung ein Hydroxylaminsalz und eine Base zum Freisetzen des Hydroxylamins aus dem Salz umfaßt.

11. Verfahren gemäß einem der Ansprüche 1-10, wobei Methylacetat oder Dimethylether zu einem Produkt carbonyliert wird, das im wesentlichen aus Essigsäureanhydrid besteht.

12. Verfahren gemäß einem der Ansprüche 1-10, wobei Methylacetat oder Dimethylether zu einem Produkt carbonyliert wird, das sowohl Essigsäure als auch Essigsäureanhydrid umfaßt.

13. Verfahren gemäß einem der Ansprüche 1-10, wobei Methanol oder Methylacetat zu einem Produkt carbonyliert wird, das im wesentlichen aus Essigsäure besteht.

14. Verfahren gemäß einem der Ansprüche 1-10, wobei Methanol oder Methylacetat zu einem Produkt carbonyliert wird, das sowohl Essigsäure als auch Essigsäureanhydrid umfaßt.

15. Verfahren gemäß einem der Ansprüche 1-14, wobei die in dem Vorlauf vorhandenen Carbonylverunreinigungen Acetaldehyd umfassen.

16. Verfahren gemäß einem der Ansprüche 1-14, wobei die in dem Vorlauf vorhandenen Carbonylverunreinigungen Ketone umfassen.

17. Verfahren gemäß Anspruch 16, wobei die Ketone Aceton beinhalten.

## Revendications

1. Un procédé de carbonylation d'un ou plusieurs composés appartenant au groupe consistant en méthanol, diméthyléther ou acétate de méthyle en un produit constitué d'acide acétique, anhydrique acétique ou de ces deux produits, dans lequel ledit méthanol, éther diméthylique ou acétate de méthyle est carbonylé dans un milieu réactionnel contenant un catalyseur de carbonylation métallique appartenant au groupe VIII de la classification périodique et de iodure de méthyle, les produits de ladite carbonylation étant séparés en une phase volatile contenant lesdits produits, en éther diméthylique ou acétate de méthyle n'ayant pas réagi et en iodure de méthyle et en une phase moins volatile renfermant ledit catalyseur métallique du groupe VIII, ladite phase volatile étant distillée pour donner un produit et une tête contenant du méthanol, de l'éther diméthylique ou de l'acétate de méthyle n'ayant pas réagi et de l'iodure de méthyle, et à recycler ladite tête dans ledit réacteur de carbonylation, l'amélioration consistant en:
mise en contact de ladite tête avec une solution aqueuse d'un dérivé aminé dans des conditions dans lesquelles ledit dérivé aminé réagit avec les impuretés carbonylées contenues dans ladite tête pour les transformer en dérivés azotés hydrosolubles desdits carbonyles, puis formation d'une phase organique contenant une proportion principale dudit éther diméthylique ou acétate de méthyle, iodure de méthyle et une phase aqueuse contenant lesdits dérivés azotés, séparation de ladite phase organique de ladite phase aqueuse pour obtenir un produit purifié recyclable dans le réacteur.

2. Procédé de carbonylation du méthanol, de l'éther diméthylique ou de l'acétate de méthyle en acide acétique, anhydride acétique ou ces deux composés, dans lequel ledit méthanol, l'éther diméthylique ou l'acétate de méthyle est carbonylé dans un milieu réactionnel contenant un catalyseur de carbonylation métallique appartenant au groupe VIII et un promoteur du catalyseur à base d'iodure organique, les produits de ladite réaction de carbonylation étant séparés en une phase volatile contenant ledit produit, le méthanol, l'éther diméthylique ou l'acétate de méthyle n'ayant pas réagi et l'iodure organique, et une phase moins volatile renfermant le catalyseur métallique du groupe VIII, la distillation de ladite phase volatile pour donner un produit et une tête renfermant le méthanol, l'éther diméthylique ou l'acétate de méthyle n'ayant pas réagi et l'iodure organique, et le recyclage de ladite tête vers ledit réacteur de carbonylation, l'améliorant consistant en:
la mise en contact de ladite tête avec un composé dans des conditions dans lesquelles ledit composé réagit avec les impuretés carbonylées dans ladite tête pour les transformer en dérivés desdits carbonylés, suivie d'une distillation de ladite tête pour séparer les têtes purifiées destinées au recyclage desdits dérivés carbonylés.

3. Procédé selon la revendication 2, dans lequel ledit composé est un dérivé aminé aqueux qui forme des dérivés azotés hydrosolubles desdits dérivés carbonylés, ledit procédé comportant en outre avant la distillation la formation d'une phase organique contenant la majeure partie desdits méthanol, éther diméthylique ou acétate de méthyle et iodure organique, et une phase aqueuse contenant lesdits dérivés azotés, la distillation de ladite phase organique pour séparer une tête purifiée dudit courant de recyclage.

4. Procédé selon la revendication 2 ou 3, dans lequel ledit promoteur de catalyseur d'iodure organique est l'iodure de méthyle.

5. Procédé selon la revendication 1, 3 ou 4, dans lequel ledit dérivé aminé est l'hydroxylamine et lesdits dérivés azotés sont des oximes.

6. Procédé selon la revendication 5, dans lequel ledit dérivé aminé comprend un sel d'hydroxylamine et une base pour libérer ladite hydroxylamine de son sel.

7. Procédé selon la revendication 6, dans lequel ledit sel d'hydroxylamine est le sulfate d'hydroxylamine.

8. Procédé selon la revendication 6 ou 7, dans lequel ladite base est l'hydroxyde de sodium.

9. Procédé selon l'une quelconque des revendications 1 à 8, dans lequel on ajoute de l'eau dans ladite séparation ou distillation de ladite phase organique pour que le rapport volumique de l'eau à la phase organique soit compris entre 0,1 et 3 de manière à diminuer la concentration en dérivés azotés dans la tête purifiée.

10. Procédé selon la revendication 9, dans lequel ladite solution aqueuse de dérivé aminé comprend un sel d'hydroxylamine et une base pour libérer ladite hydroxylamine dudit sel.

11. Procédé selon l'une quelconque des revendications 1 à 10, dans lequel l'acétate de méthyle ou l'éther diméthylique est carbonylé en un produit consistant essentiellement en anhydride acétique.

12. Procédé selon l'une quelconque des revendications 1 à 10, dans lequel l'acétate de méthyle ou l'éther diméthylique est carbonylé en un produit contenant à la fois de l'acide acétique et de l'anhydride acétique.

13. Procédé selon l'une quelconque des revendications 1 à 10, dans lequel le méthanol ou l'acétate de méthyle est carbonylé en un produit consistant essentiellement en acide acétique.

14. Procédé selon l'une quelconque des revendications 1 à 10, dans lequel le méthanol ou l'acétate de méthyle est carbonylé en un produit comprenant à la fois de l'acide acétique et de l'anhydride acétique.

15. Procédé selon l'une quelconque des revendications 1 à 14, dans lequel lesdites impuretés carbonylées présentes dans ladite tête renferment de l'acétaldéhyde.

16. Procédé selon l'une quelconque des revendications 1 à 14, dans lequel lesdites impuretés carbonylées présentes dans lesdites têtes renferment des cétones.

17. Procédé selon la revendication 16, dans lequel lesdites cétones renferment de l'acétone.
